Europäisches Patentamt

European Patent Office

Office européen des brevets

(11) Veröffentlichungsnummer: **0 298 233 B1**

(12) **EUROPÄISCHE PATENTSCHRIFT**

(45) Veröffentlichungstag der Patentschrift: 27.12.91

(51) Int. Cl.⁵: **A61F 2/44**

(21) Anmeldenummer: 88108214.3

(22) Anmeldetag: 21.05.88

(54) Metallische Zwischenwirbel-Prothese.

(30) Priorität: 09.07.87 CH 2605/87

(43) Veröffentlichungstag der Anmeldung:
11.01.89 Patentblatt 89/02

(45) Bekanntmachung des Hinweises auf die
Patenterteilung:
27.12.91 Patentblatt 91/52

(84) Benannte Vertragsstaaten:
AT DE FR GB IT

(56) Entgegenhaltungen:
EP-A- 0 179 695
DE-A- 2 263 842
DE-A- 2 365 873
US-A- 3 867 728

(73) Patentinhaber: **GEBRÜDER SULZER AKTIENGE-
SELLSCHAFT**
**Zürcherstrasse 9**
**CH-8401 Winterthur(CH)**

(72) Erfinder: **Frey, Otto**
**Wallrütistrasse 56**
**CH-8400 Winterhur(CH)**
Erfinder: **Koch, Rudolf**
**Oberdorfstrasse 229**
**CH-8267 Berlingen(CH)**

**Beschreibung**

Die Erfindung betrifft eine metallische Zwischenwirbel-Prothese für eine Arthrodese.

Für eine Versteifung (Arthrodese) zweier Lendenwirbel relativ zueinander sind bisher zwei unterschiedliche Vorgehensweisen üblich. Bei dem einen Verfahren werden an anderen Stellen des Körpers, beispielsweise aus dem Becken oder dem Wadenbein, entnommene Knochensplitter als teilweiser Ersatz für die beschädigten Bandscheiben zwischen zwei Wirbeln von ventral her eingesetzt und mit Hilfe von Knochenschrauben fixiert. Es hat sich gezeigt, dass - vor allem kurz nach der Implantation, wenn das Knochengewebe noch nicht mit den Wirbelkörpern verwachsen ist - die Fixierung der beiden angrenzenden Wirbel ungenügend ist und keine ausreichende Stabilität besitzt.

Bei dem anderen Verfahren werden zwei benachbarte Wirbel durch kreuzförmige Platten, die von dorsal her mit den beiden Wirbelkörpern, beispielsweise ebenfalls durch Schrauben, fest verbunden werden, relativ zueinander fixiert. Auch hier hat die Praxis gezeigt, dass die relativ weit von den Bandscheiben entfernt angeordneten Kreuzplatten im dorsalen Bereich der Wirbel - besonders bei grösseren Bandscheibenschäden oder bei einem kompletten Ersatz einer Bandscheibe - im Bereich der Bandscheiben nur eine ungenügende Fixierung der Wirbel bewirken und ebenfalls zu Instabilitäten in der gegenseitigen Versteifung der Wirbel neigen.

EP-A 0 179 695 zeigt eine Platte mit Befestigungsbohrungen, zu der eine Zwischenscheibe konstanter Dicke im rechten Winkel balkonartig wegsteht. Das Einbringen der Zwischenscheibe und das Herstellen einer Primärverankerung, bei der die Zwischenscheibe trägt, stellt hier erhöhte Anforderungen an den Operateur.

Aufgabe der Erfindung ist es daher, eine mit relativ geringem operativen Aufwand implantierbare und fixierbare Zwischenwirbelprothese zu schaffen, mit der als Bandscheibenersatz feste und stabile Versteifung zweier Wirbel erreicht wird. Mit der vorliegenden Erfindung wird dieses Ziel dadurch erreicht, dass sie aus einem scheibenartigen Prothesenkörper besteht, an dessem ventral gelegenen Rand mit Durchtrittsöffnungen für Knochenschrauben versehene Laschen angesetzt sind, und dass sich die Dicke des Prothesenkörpers vom ventral nach dorsal konisch verjüngt.

Die neue Prothese zeichnet sich durch grosse Einfachheit aus, da sie lediglich aus einer die Bandscheibe ersetzenden Platte besteht. Ihre Primärfixierung erfolgt mit Knochenschrauben, die von ventral in die Wirbelkörper eingeschraubt werden, nachdem der Prothesenkörper ebenfalls von ventral in den zuvor von der zerstörten Bandscheibe geräumten Zwischenraum zwischen den Wirbeln eingeschoben worden ist.

Für die Anpassung der Prothese an die anatomischen Gegebenheiten ist es vorteilhaft, wenn sich die Dicke des Prothesenkörpers von ventral nach dorsal konisch verjüngt und wenn der Prothesenkörper nierenförmig ausgebildet ist.

Für die Langzeitfixierung ist es vorteilhaft, wenn die Scheibenoberflächen des Prothesenkörpers mit einer Struktur für das Ein- und/oder Anwachsen von Gewebe versehen sind. Insbesondere hat sich dabei als Struktur ein mindestens einlagiges Netz aus Metalldraht bewährt, das beispielsweise durch über die Fläche verteilte Punktschweissungen auf den Scheibenoberflächen des Prothesenkörpers gehalten ist.

Geeignete Werkstoffe für die Herstellung der neuen Prothese sind die in der Implantat-Technik verwendeten Metalle und Metall-Legierungen; bevorzugt werden Titan und Titan-Legierungen.

Im folgenden wird die Erfindung anhand von Ausführungsbeispielen im Zusammenhang mit der Zeichnung näher erläutert.

Fig. 1    ist eine Aufsicht auf einen ersten Prothesenköroper;

Fig. 2    stellt eine Ansicht von Fig. 1 von links dar;

Fig. 3    ist eine Ansicht eines eingesetzten Implantates von ventral;

Fig. 4    gibt in gleicher Darstellung wie Fig. 1 ein zweite Ausführungsform der Erfindung wieder, während

Fig. 5    der Schnitt V-V von Fig. 4 ist.

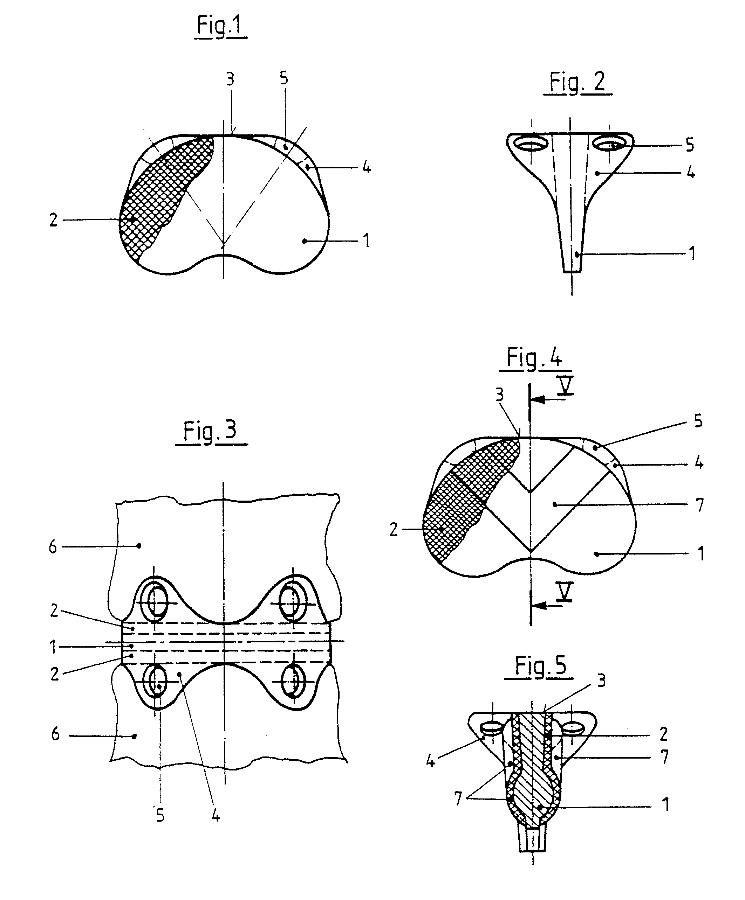

Der scheibenartige Prothesenkörper 1, der ventral konvex und dorsal konkav gekrümmt ist, hat eine von ventral nach dorsal abnehmende Dicke (Fig. 2). Er ist auf seinen Scheibenoberflächen mit einer Struktur 2 versehen, die im vorliegenden Beispiel aus einem einlagigen Drahtnetz besteht; dieses ist durch über die nierenförmige Scheibenoberfläche verteilte Punktschweissungen befestigt.

Am konvex gekrümmten ventral gelegenen Rand 3 des Prothesenkörpers 1 sind symmetrisch zu seiner Mittelebene aus der Scheibenfläche nach oben und unten hervorstehende Laschen 4 angesetzt, die je eine Bohrung 5 für den Durchtritt einer nicht gezeigten Knochenschraube haben. Die Bohrungen 5 sind als Teil einer Kugelschale ausgeführt, um eine Ausichtung der Schrauben in eine Linie zu erleichtern.

Nach dem Ausführungsbeispiel in Fig. 4, das im sonstigen Aufbau demjenigen nach Fig. 1 - 3 entspricht, ist auf jeder Scheibenoberfläche des Prothesenkörpers 1 eine wulstartige Erhebung 7 vorgesehen, die die Querstabilität der Versteifung erhöht.

Wie in Fig. 3 angedeutet, schmiegen sich die Laschen 4 weitgehend an die Wirbelkörper 6 an, in die zur Primärfixierung der Prothese Knochenschrauben eingeschraubt werden.

**Patentansprüche**

1. Metallische Zwischenwirbel-Prothese für eine Arthrodese, die aus einem scheibenartigen Prothesenkörper (1) besteht, an dessem ventral gelegenen Rand (3) mit Durchtrittsöffnungen (5) für Knochenschrauben versehene Laschen (4) angesetzt sind, dadurch gekennzeichnet, dass sich die Dicke des Prothesenkörpers (1) von ventral nach dorsal konisch verjüngt.

2. Zwischenwirbel-Prothese nach Anspruch 1, dadurch gekennzeichnet, dass der Prothesenkörper (1) nierenförmig ausgebildet ist.

3. Zwischenwirbel-Prothese nach Anspruch 1 oder 2, dadurch gekennzeichnet, dass die Scheibenoberflächen des Prothesenkörpers (1) mit einer Struktur (2) für das Ein- und/oder Anwachsen von Gewebe versehen sind.

4. Zwischenwirbel-Prothese nach Anspruch 3, dadurch gekennzeichnet, dass die Struktur (2) aus einem mindestens einlagigen Netz aus Metalldraht besteht.

**Claims**

1. A metal intervertebral prosthesis for an arthrodesis, the prosthesis consisting of a discoid prosthesis body (1), tabs (4) which are formed with passages (5) for bone screws being devised on the ventral edge (3) of the body (1), characterised in that the thickness of the prosthesis body (1) narrows conically ventrally to dorsally.

2. A prosthesis according to claim 1, characterised in that the prosthesis body (1) is reniform.

3. A prosthesis according to claim 1 or 2, characterised in that the disc surfaces of the prosthesis body (1) have a structure (2) promoting the invasion and/or ongrowth of tissue.

4. A prosthesis according to claim 3, characterised in that the structure (2) comprises at least one single-layer metal wire mesh.

**Revendications**

1. Prothèse métallique de disque intervertébral pour une arthrodèse, qui est constituée d'un corps de prothèse (1) en forme de disque sur le bord (3) ventral duquel sont placées des pattes (4) pourvues d'orifices de passage (5) pour des vis à os, caractérisée en ce que l'épaisseur du corps de prothèse (1) se rétrécit coniquement du côté ventral vers le côté dorsal.

2. Prothèse de disque intervertébral selon la revendication 1, caractérisée en ce que le corps de prothèse (1) est réniforme.

3. Prothèse de disque intervertébral selon la revendication 1 ou 2, caractérisée en ce que les surfaces discales du corps de prothèse (1) sont pourvues d'une structure (2) pour une croissance du tissu en profondeur et/ou en surface.

4. Prothèse de disque intervertébral selon la revendication 3, caractérisée en ce que la structure (2) est constituée par une grille à au moins une couche de fil métallique.

Fig. 1

Fig. 2

Fig. 3

Fig. 4

Fig. 5